# FASCICULE DE BREVET EUROPEEN

(11) **EP 0 451 300 B1**
(45) Date de publication et mention de la délivrance du brevet: **26.10.1994**
(21) Numéro de dépôt: 87202259.5
(22) Date de dépôt: 10.04.1990
(51) Int. Cl.: A61K 33/04, A61K 7/48

(54) **Composition contenant une dispersion colloidale de sulfate de baryum**
Bariumsulfat-kolloidale Dispersion enthaltende Zusammensetzung
Composition containing a colloidal barium sulphate dispersion

(43) Date de publication de la demande: 16.10.1991
(73) Titulaire: Kowalski, Romuald, Dr., Matejki (PL)
(72) Inventeur: Kowalski, Romuald, Dr., Matejki (PL)
(74) Mandataire: Roman, Michel

(56) Documents cités:
- FR-A- 2 639 829
- US-A- 4 477 439
- CHEMICAL ABSTRACTS,vol. 100, no. 22, 28 mai 1984, Columbus, OH (US); P.A.WILLIAMS et al., p. 399, no. 198245h #
- Chemical Abstracts, vol. 97, no.10, 6 Septembre 1982, Columbus, OhioUSA, Williams P.A. et Al. page467, colonne 1; ref no. 79491V

## Description

En résultat des expériences faites, on a réussi à obtenir une substance active à base de sulfate de baryum en dispersion colloïdale sous la forme d'une masse pareille à la roche, appelée "Laitvit - Sulfate R.K." simple.

Cette substance, soumise à une nouvelle trituration pour la réduire en poudre constitue la substance utilisée pour la production de la crème dont le nom est "Crème - Laitvit - R.K." simple.

Cette substance est atoxique, son action est douce et elle ne subit pas une décomposition rapide.

Jusqu'à présent seul l'usage à des fins diagnostiqués de suspensions colloïdales de sulfate de baryum étant connu dans l'art antérieur.

Pour la production de cette crème on utilise pour la substance active du sulfate de baryum chimiquement pur, aussi bien que du sulfate de baryum provenant du minerai de même nom. On obtient la "crème - Laitvit - mineral - R.K.", dans la composition de laquelle entre la substance chimique obtenue directement à partir du minerai sulfate de baryum qui, privé en partie importante des composés chimiques accompagnant ce minérai et ensuite soumise à la dispersion colloïdale, à une protection convenable contre une désintégration prématurée, après être conservée convenablement et désséchée, est triturée en poudre dite "Laitvit - sulfate - minéral R.K." en poudre servant à la confection de la "crème - Laitvit - minéral - R.K." (voir les formules N° 1 et 2).

L'index graphique des substances actives entrant dans la composition de la crème "Laitvit - R.K." est montré par le schéma N° 1.

| FORMULE N° 1: | |
|---|---|
| - Sulfate de baryum chimiquement pur | 100,0 g |
| - Pectine | 1,0 g |
| - Carboxymethyl cellulose,sel de sodium | 0,5 g |
| - Acide citrique | 0,5 g |
| - Carbonate de calcium | 0,1 g |

| Conservateurs: | |
|---|---|
| - hydroxybenzoate de propyle | 0,1 g |
| - hydrobenzoate de méthyle | 0,1 g |
| - Sorbate de potassium | 0,3 g |
| - Eau distillée q.s.p. 100 ml | |

| FORMULE N° 2: | |
|---|---|
| - Sulfate de baryum-Minéral 98% SiO2 1,02% / Fe2O3 0,3% / H20 0,1% | 100,0 g |
| - Pectine | 1,0 g |
| - Acide citrique | 0,5 g |

| Conservateurs: | |
|---|---|
| - hydroxybenzoate de propyle | 0,1 g |
| - hydroxybenzoate de méthyle | 0,1 g |
| - Sorbate de potassium | 0,1 g |
| - Carboxymethylocellulose, Sel de sodium | 0,5 g |
| - Eau distillée q.s.p. 100 ml | |

Index graphique des substances actives entrant dans la composition de la crème "LAITVIT - R.K." 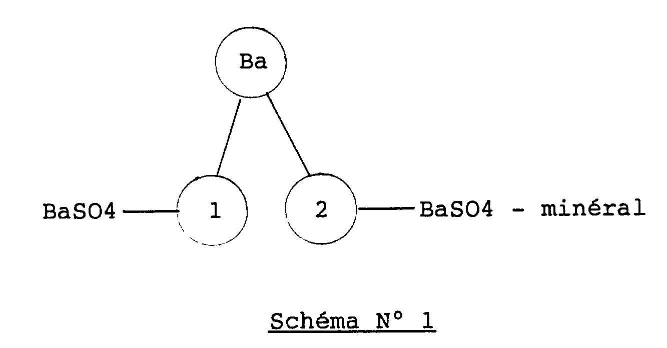
1: Crème "Laitvit - R.K." dans la composition de laquelle entre la substance active à base de sulfate de baryum chimiquement pur, soumise à une dispersion colloïdale.
2: Crème "Laitvit - minéral R.K." dans la composition de laquelle entre la substance active à base de sulfate de baryum obtenu directement à partir du minérai du même nom, soumise à une dispersion colloïdale.

Selon la teneur en pourcentage de cette substance active et aussi selon le véhicule avec lequel cette substance est mélangée, on obtient les crèmes suivantes:
1. La crème "Laitvit - d'usage général R.K."
2. La crème prophylactique "Laitvit - jeunesse - R.K."
3. La crème "Laitvit - oculaire - R.K."
4. La crème "Laitvit - bébé - R.K."
5. La crème "Laitvit - beauté - R.K."

1.- La crème "Laitvit - d'usage général R.K." est préparée selon la formule N° 1G (voir la formule N° 1G). Dans la composition de cette crème entre aussi la substance active "Sulfate - minéral - R.K." qui par voie de dispersion colloïdale a été affaiblie dans son action. Elle est devenue douce dans son action et atoxique pour les hommes et les animaux (voir la formule 2G).
2.- La crème prophylactique "Laitvit - jeunesse - R.K." est préparée selon la formule N° 2J (voir la formule N° 2J).
3.- La crème "Laitvit - oculaire - R.K." est préparée selon la formule N° 30 (voir la formule N° 30).
4.- La crème Laitvit - bébé - R.K." est préparée selon la formule N° 4B (voir la formule N° 4B).
5.- La crème "Laitvit - beauté - R.K." est préparée selon la formule N° 5B (voir la formule N° 5B).

Il est également possible de formuler une crème à raser ou une crème liquide à partir de laitvit R.K.

### Crème "Laitvit - d'usage général R.K."

| Formule chimique N° 1G | |
|---|---|
| - "Laitvit - Sulfate R.K." | 1,0 - 2,0 g |
| - Céresine | 9,0 g |
| - Alcool cétylique | 0,5 g |
| - Huile de paraffine et vaseline blanche | 22,0 g |
| - Glycérine | 2,0 g |
| - Paraffine | 1,0 - 2,0 g |
| - Stéarate de magnésium | 0,5 g |
| - Sulfate de magnésium -sulfate de magnésie | 0,5 g |
| - Acide citrique | 0,6 g - 50% |
| - Blanc de zinc | 0,2 g |
| - Antioxydant | 0,1% |
| - Composé / arôme | 0,15% |
| - Eau distillée q.s.p. 100 ml | |

### Crème "Laitvit - minéral - d'usage général R.K."

| Formule chimique N° 2G: | |
|---|---|
| - "Laitvit - sulfate - minéral R.K." | 1,0 - 2,0 g |
| - Céresine | 9,0 g |
| - Alcool cétylique | 0,5 g |
| - Huile de paraffine et vaseline blanche | 22,0 g |
| - Glycérine | 2,0 g |
| - Paraffine | 1,0 - 2,0 g |
| - Stéarate de magnésium | 0,5 g |
| - Sulfate de magnésie | 0,5 g |
| - Acide citrique | 0,5 g - 50% |
| - Blanc de zinc | 0,2 g |
| - Antioxydant | 0,1 g |
| - Composé / arôme | 0,15 g |
| - Eau distillée q.s.p. 100 ml | |

### Crème prophylactique "Laitvit-jeunesse -R.K."

| Formule chimique N° 2J: | |
|---|---|
| - "Laivit - sulfate - R.K." | 1,5 g |
| - Vaseline blanche | 40,0 g |
| - Glycérine | 40,0 g |
| - Lanoline | 10,0 g |
| - Alcool cétylique | 0,5 g |
| - Acide citrique | 0,5 g - 50% |
| - Blanc de zinc | 0,2 g |
| - Antioxydant | 0,1 g |
| - Composé / arôme | 0,15 g |
| - Vitamine A | 8000 u.i. |
| - Eau distillée q.s.p. 100 ml | |

### Crème "Laitvit - oculaire - R.K."

| Formule chimique N° 3O: | |
|---|---|
| - "Laivit - sulfate - R.K." | 0,75 g |
| - Vaseline blanche | 40,0 g |
| - Glycérine | 40,0 g |
| - Lanoline | 5,0 g |
| - Acide citrique | 0,05 g- 50% |
| - Antioxydant | |
| - Eau distillée q.s.p. 100 ml | |

### Crème "Laitvit - bébé - R.K."

| Formule chimique N° 4B: | |
|---|---|
| - "Laivit - sulfate - R.K." | 1,25 g |
| - Céresine | 8,0 g |
| - Huile de paraffine | 25,0 g |
| - Vaseline blanche | 25,0 g |
| - Glycérine | 5,0 g |
| - Lanoline | 2,0 g |
| - Talc | 10,0 g |
| - Vitamine A | 8000 u.i |
| - Antioxydant | 0,1 g |
| - Composé / arôme | 0,15 g |
| - Eau distillée q.s.p. 100 ml | |

### Crème "Laitvit - beauté - R.K."

| Formule chimique N° 5B: | |
|---|---|
| - "Laivit - sulfate - R.K." | 1,0 g |
| - Vaseline blanche | 45,0 g |
| - Glycérine | 45,0 g |
| - Lanoline | 5,0 g |
| - Acide citrique | 0,05 g - 50% |
| - Antioxydant | 0,01 g |
| - Vitamine E | |
| - Composé / arôme | 0,15 g |
| - Eau distillée q.s.p. 100 ml | |

La substance active "Laitvit - sulfate - R.K." simple, grâce au sulfate de baryum entrant dans sa composition possède des propriétés:
- anti-inflammatoires,
- analgésiques,
- antipruritiques,
- antiallergiques,
- antibactériennes,
- antivirales et antimycotiques,
ainsi que des propriétés améliorant la vascularisation de la peau et certaine propriétés de blanchiment de celle-ci.

## Revendications

1. Utilisation d'une composition contenant une dispersion colloidale de sulfate de baryum, obtenue à partir d'une formulation à base de sulfate de baryum préalablement mise en dispersion colloidale, séchée et réduite en poudre, préparer un médicament à usage médical prophylactique ou thérapeutique.

2. Composition active de base constituée de:
| | |
|---|---|
| - Sulfate de baryum chimiquement pur | 100,0 g |
| - Pectine | 1,0 g |
| - Carboxyméthylocellulose, sel de sodium | 0,5 g |
| - Acide citrique | 0,5 g |
| - Carbonate de calcium | 0,1 g |
| Conservateurs: | |
|---|---|
| - hydroxybenzoate de propyle | 0,1 g |
| - hydroxybenzoate de méthyle | 0,1 g |
| - Sorbate de potassium | 0,3 g |
| - Eau distillée q;s;p; 100 ml. | |

3. Composition active de base constituée de:
| | |
|---|---|
| - Minerai de sulfate de baryum partiellement épuré contenant 98% de sulfate de baryum minéral, 1,02% de SiO2, 0,3% de Fe2O3 et 0,1% de H2O | 100,0 g |
| - Pectine | 1,0 g |
| - Acide citrique | 0,5 g |
| Conservateurs: | |
|---|---|
| - Hydroxybenzoate de propyle | 0,1 g |
| - hydroxybenzoate de méthyle | 0,1 g |
| - Sorbate de potassium | 0,1 g |
| - Carboxymethylocellulose, sel de sodium | 0,5 g |
| - Eau distillée q.s.p. 100 ml | |

4. Crème d'usage général se caractérisant par le fait qu'elle est composée de:
| | |
|---|---|
| - Substance active à base de sulfate de baryum, en poudre, selon la revendication 2 ou 3 | 1,0 - 2,0 g |
| - Céresine | 9,0 g |
| - Alcool cetylique | 0,5 g |
| - Huile de paraffine et vaseline blanche | 22,0 g |
| - Glycérine | 2,0 g |
| - Paraffine | 1,0 - 2,0 g |
| - Stéarate de magnésium | 0,5 g |
| - Sulfate de magnésium-sulfate de magnésie | 0,5 g |
| - Acide citrique | 0,6 g - 50 % |
| - Blanc de zinc | 0,2 g |
| - Antioxydant | 0,1 % |
| - Composé / arôme | 0,15% |
| - Eau distillée q.s.p. 100 ml | |

5. Crème prophylactique se caractérisant par le fait qu'elle est composée de:
| | |
|---|---|
| - Substance active à base de sulfate de baryum, en poudre, selon la revendication 2 ou 3 | 1,5 g |
| - Vaseline blanche | 40,0 g |
| - Glycérine | 40,0 g |
| - Lanoline | 10,0 g |
| - Alcool cetylique | 0,5 g |
| - Acide citrique | 0,5 g - 50% |
| - Blanc de zinc | 0,2 g |
| - Antioxydant | 0,1 g |
| - Composé / arôme | 0,15 g |
| - Vitamine A | 8000 u.i. |
| - Eau distillée q.s.p. 100 ml | |

6. Crème oculaire se caractérisant par le fait qu'elle est composée de:
| | |
|---|---|
| - Substance active à base de sulfate de baryum, en poudre, selon la revendication 2 ou 3 | 0,75 g |
| - Vaseline blanche | 40,0 g |
| - Glycérine | 40,0 g |
| - Lanoline | 5,0 g |
| - Acide citrique | 0,05 g - 50% |
| - Antioxydant | |
| - Eau distillée q.s.p. 100 ml | |

7. Crème pour bébés se caractérisant par le fait qu'elle est composée de:
| | |
|---|---|
| - Substance active à base de sulfate de baryum, en poudre, selon la revendication 2 ou 3 | 1,25 g |
| - Céresine | 8,0 g |
| - Huile de paraffine | 25,0 g |
| - Vaseline blanche | 25,0 g |
| - Glycérine | 5,0 g |
| - Lanoline | 2,0 g |
| - Talc | 10,0 g |
| - Vitamine A | 8000 u.i. |
| - Antioxydant | 0,1 g |
| - Composé / arôme | 0,15 g |
| - Eau distillée q.s.p. 100 ml | |

8. Crème de beauté se caractérisant par le fait qu'elle est composée de:
| | |
|---|---|
| - Substance active à base de sulfate de baryum, en poudre, selon la revendication 2 ou 3 | 1,0 g |
| - Vaseline blanche | 45,0 g |
| - Glycérine | 45,0 g |
| - Lanoline | 5,0 g |
| - Acide citrique | 0,05 g - 50% |
| - Antioxydant | 0,01 g |
| - Vitamine E | |
| - Composé / arôme | 0,15 g |
| - Eau distillée q.s.p. 100 ml | |

9. Crème à raser se caractérisant par le fait qu'elle contient de la substance active à base de sulfate de baryum, en poudre, selon l'une des revendications 2 ou 3 .

10. Utilisation d'une substance active obtenue à partir d'une formulation à base de sulfate de baryum préalablement mise en dispersion colloidale, séchée et réduite en poudre pour la fabrication d'un analgésique.

11. Utilisation d'une substance active obtenue à partir d'une formulation à base de sulfate de baryum préalablement mise en dispersion colloidale, séchée et réduite en poudre pour la fabrication d'une composition anti-pruritique.

12. Utilisation d'une substance active obtenue à partir d'une formulation à base de sulfate de baryum préalablement mise en dispersion colloidale, séchée et réduite en poudre pour la fabrication d'un médicament pour traiter les allergies.

13. Utilisation d'une substance active obtenue à partir d'une formulation à base de sulfate de baryum préalablement mise en dispersion colloidale, séchée et réduite en poudre pour la fabrication d'une composition antibactérienne.

14. Utilisation d'une substance active obtenue à partir d'une formulation à base de sulfate de baryum préalablement mise en dispersion colloidale, séchée et réduite en poudre pour la fabrication d'une composition antivirale et antimycotique.

15. Utilisation d'une substance active obtenue à partir d'une formulation à base de sulfate de baryum préalablement mise en dispersion colloidale, séchée et réduite en poudre pour la fabrication d'une composition pour améliorer la vascularisation de la peau.

16. Utilisation d'une substance active obtenue à partir d'une formulation à base de sulfate de baryum préalablement mise en dispersion colloidale, séchée et réduite en poudre pour la fabrication d'une composition pour le blanchiment de la peau.

## Claims

1. Use of a composition containing a colloidal dispersion of barium sulfate obtained from a formula with a barium sulfate base, previously placed in colloidal dispersion, dried and reduced to powder, in order to prepare a medical preparation for profilactic and therapeutic medicine.

2. Active composition of the base consisting of:
| | |
|---|---|
| - Chemically pure barium sulfate | 100.0 g |
| - Pectin | 1.0 g |
| - Carboxymethylocellulose, sodium salt | 0.5 g |
| - Citric acid | 0.5 g |
| - Calcium carbonate | 0.1 g |
| Preserving agents: | |
|---|---|
| - Propylhydroxybenzoate | 0.1 g |
| - Methylhydroxybenzoate | 0.1 g |
| - Potassium sorbate | 0.3 g |
| - Distilled water q.s. to 100 ml | |

3. Active composition of the base consisting of:
| | |
|---|---|
| - Mineral barium sulfate, partially purified, containing 98% mineral barium sulfate, 1.02% SiO2, 0.3% Fe2O3 and 0.1% of H2O | 100.0 g |
| - Pectin | 1.0 g |
| - Citric acid | 0.5 g |
| Preserving agents: | |
|---|---|
| - Propylhydroxybenzoate | 0.1 g |
| - Methylhydroxybenzoate | 0.1 g |
| - Potassium sorbate | 0.3 g |
| - Carboxymethylocellulose, sodium salt | 0.5 g |
| - Distilled water q.s. to 100 ml | |

4. General purpose cream characterized by the fact that its composition is:
| | |
|---|---|
| - Active substance with barium sulfate base, in powder form, as per claim 2 or 3 | 1.0 - 2.0 g |
| - Ceresine | 9.0 g |
| - Cetylic alcohol | 0.5 g |
| - Paraffin oil and white vaseline | 22.0 g |
| - Glycerin | 2.0 g |
| - Magneso stearate | 0.5 g |
| - Magnesium sulfate - Sulfate of magnesia | 0.5 g |
| - Citric acid | 0.6 g - 50 % |
| - Zinc white | 0.2 g |
| - Antioxydizing agent | 0.1 % |
| - Aroma/compound | 0.15% |
| - Distilled water, q.s. to 100 ml | |

5. Prophylactic cream characterized by the fact that its composition is:
| | |
|---|---|
| - Active substance with barium sulfate, in powder form, as per claims 2 or 3 | 1.5 g |
| - White vaseline | 40.0 g |
| - Glycerine | 40.0 g |
| - Lanolin | 10.0 g |
| - Cetylic alcohol | 0.5 g |
| - Citric acid | 0.5 g - 50 % |
| - Zinc white | 0.2 g |
| - Antioxydizing agent | 0.1 g |
| - Aroma/compound | 0.15g |
| - Vitamin A | 8000 i.u. |
| - Distilled water, q.s. to 100 ml | |

6. Ocular cream characterized by the fact that its composition is:
| | |
|---|---|
| - Active substance with barium sulfate base, in powder form, as per claim 2 or 3 | 0.75 g |
| - White vaseline | 40.0 g |
| - Glycerine | 40.0 g |
| - Lanolin | 5.0 g |
| - Citric acid | 0.5 g - 50 % |
| - Antioxydizing agent | |
| - Distilled water, q.s. to 100 ml | |

7. Baby cream characterized by the fact that its composition is:
| | |
|---|---|
| - Active substance with barium sulfate base, in powder form, as per claim 2 or 3 | 1.25 g |
| - Ceresine | 8.0 g |
| - Paraffin oil | 25.0 g |
| - White vaseline | 25.0 g |
| - Glycerine | 5.0 g |
| - Lanolin | 2.0 g |
| - Talcum powder | 10.0 g |
| - Vitamin A | 8000 i.u. |
| - Antioxydizing agent | 0.1 % |
| - Aroma/compound | 0.15% |
| - Distilled water, q.s. to 100 ml | |

8. Beauty cream characterized by the fact that its composition is:
| | |
|---|---|
| - Active substance with barium sulfate base, in powder form, as per claim 2 or 3 | 1.0 g |
| - White vaseline | 45.0 g |
| - Glycerine | 45.0 g |
| - Lanolin | 5.0 g |
| - Citric acid | 0.05 g - 50% |
| - Antioxydizing agent | 0.01% |
| - Vitamin E | |
| - Aroma/compound | 0.15% |
| - Distilled water, q.s. to 100 ml | |

9. Shaving cream characterized by the fact that it contains the active substance with barium sulfate base, in powder form, as per one of the claims 2 or 3.

10. Use of an active substance obtained from a formulation with barium sulfate base previously subject to colloidal dispersion, dried and reduced to powder form to manufacture an analgesic.

11. Use of an active substance obtained from a formulation with barium sulfate base previously subject to colloidal dispersion, dried and reduced to powder form to manufacture an anti-pruritic composition.

12. Use of an active substance obtained from a formulation with barium sulfate base previously subject to colloidal dispersion, dried and reduced to powder form to manufacture a medecine for the treatment of allergies.

13. Use of an active substance obtained from a formulation with barium sulfate base previously subject to colloidal dispersion, dried and reduced to powder form to manufacture an anti-bacterial composition

14. Use of an active substance obtained from a formulation with barium sulfate base previously subject to colloidal dispersion, dried and reduced to powder form to manufacture an anti-viral and anti-mycotic composition.

15. Use of an active substance obtained from a formulation with barium sulfate base previously subject to colloidal dispersion, dried and reduced to powder form to manufacture a composition for improving skin vascularisation.

16. Use of an active substance obtained from a formulation with barium sulfate base previously subject to colloidal dispersion, dried and reduced to powder form to manufacture a composition for whitening the skin.

## Patentansprüche

1. Verwendung einer Zusammensetzung, die eine Bariumsulfat-Kolloiddispersion enthält, erzeugt anhand einer Formulierung auf der Grundlage von zuvor in Kolloiddispersion gebrachtem, getrocknetem und zu Pulver reduziertem Bariumsulfat zur Bereitung eines Medikaments für prophylaktische oder therapeutische medizinische Anwendung.

2. Aktive Grundzusammensetzung, bestehend aus:
| | |
|---|---|
| - chemisch reinem Bariumsulfat | 100,0 g |
| - Pektin | 1,0 g |
| - Carboxymethylozellulose, Natriumsalz | 0,5 g |
| - Zitronensäure | 0,5 g |
| - Kalziumkarbonat | 0,1 g |
| Konservatoren: | |
|---|---|
| - Propylradikal-Hydroxybenzoat | 0,1 g |
| - Methyl-Hydroxybenzoat | 0,1 g |
| - Kaliumsorbat | 0,3 g |
| - destilliertes Wasser für 100 ml. | |

3. Aktive Grundzusammensetzung, bestehend aus:
| | |
|---|---|
| - teilweise gereinigtem Bariumsulfaterz mit 98 % mineralischem Bariumsulfat, 1,02 % SiO2, 0,3 % Fe2O3 und 0,1 % H2O | 100,0 g |
| - Pektin | 1,0 g |
| - Zitronensäure | 0,5 g |
| Konservatoren: | |
|---|---|
| - Propylradikal-Hydroxybenzoat | 0,1 g |
| - Methyl-Hydroxybenzoat | 0,1 g |
| - Kaliumsorbat | 0,1 g |
| - Carboxymethylozellulose, Natriumsalz | 0,5 g |
| - destilliertes Wasser für 100 ml. | |

4. Creme für allgemeine Anwendung, gekennzeichnet durch ihre Bestandteile:
| | |
|---|---|
| - Aktive Substanz auf Bariumsulfatbasis, in Pulverform, gemäß Anspruch 2 oder 3 | 1,0 - 2,0 g |
| - Ceresin | 9,0 g |
| - Ketylalkohol | 0,5 g |
| - Paraffinöl und weißes Vaselin | 22,0 g |
| - Glyzerin | 2,0 g |
| - Paraffin | 1,0 - 2,0 g |
| - Magnesiumstearat | 0,5 g |
| - Magnesiumsulfat - Magnesiasulfat | 0,5 g |
| - Zitronensaure | 0,6 g - 50 % |
| - Zinkweiß | 0,2 g |
| - Oxydationsinhibitor | 0,1 % |
| - Aroma-Verbindung | 0,15 % |
| - destilliertes Wasser für 100 ml. | |

5. Prophylaktische Creme, gekennzeichnet durch ihre Bestandteile:
| | |
|---|---|
| - Aktive Substanz auf Bariumsulfatbasis, in Pulverform, gemäß Anspruch 2 oder 3 | 1,5 g |
| - Weißes Vaselin | 40,0 g |
| - Glyzerin | 40,0 g |
| - Lanolin | 10,0 g |
| - Ketylalkohol | 0,5 g |
| - Zitronensäure | 0,5 g - 50 % |
| - Zinkweiß | 0,2 g |
| - Oxydationsinhibitor | 0,1 g |
| - Aroma-Verbindung | 0,15 g |
| - Vitamin A | 8000 i.E. |
| - destilliertes Wasser für 100 ml. | |

6. Augencreme, gekennzeichnet durch ihre Bestandteile:
| | |
|---|---|
| - Aktive Substanz auf Bariumsulfatbasis, in Pulverform, gemäß Anspruch 2 oder 3 | 0,75 g |
| - Weißes Vaselin | 40,0 g |
| - Glyzerin | 40,0 g |
| - Lanolin | 5,0 g |
| - Zitronensäure | 0,05 g - 50 % |
| - Oxydationsinhibitor | |
| - destilliertes Wasser für 100 ml. | |

7. Babycreme, gekennzeichnet durch ihre Bestandteile:
| | |
|---|---|
| - Aktive Substanz auf Bariumsulfatbasis, in Pulverform, gemäß Anspruch 2 oder 3 | 1,25 g |
| - Ceresin | 8,0 g |
| - Paraffinöl | 25,0 g |
| - weißes Vaselin | 25,0 g |
| - Glyzerin | 5,0 g |
| - Lanolin | 2,0 g |
| - Talk | 10,0 g |
| - Vitamin A | 8000 i.E. |
| - Oxydationsinhibitor | 0,1 g |
| - Aroma-Verbindung | 0,15 g |
| - destilliertes Wasser für 100 ml. | |

8. Schönheitscreme, gekennzeichnet durch ihre Bestandteile:
| | |
|---|---|
| - Aktive Substanz auf Bariumsulfatbasis, in Pulverform, gemäß Anspruch 2 oder 3 | 1,0 g |
| - Weißes Vaselin | 45,0 g |
| - Glyzerin | 45,0 g |
| - Lanolin | 5,0 g |
| - Zitronensäure | 0,05 g - 50 % |
| - Oxydationsinhibitor | 0,01 g |
| - Vitamin E | |
| - Aroma-Verbindung | 0,15 g |
| - destilliertes Wasser für 100 ml. | |

9. Rasiercreme, dadurch gekennzeichnet, daß sie eine aktive Substanz auf der Basis von Bariumsulfat, in Pulverform, gemäß einem der Ansprüche 2 oder 3 enthält.

10. Verwendung einer aktiven Substanz, erzeugt anhand einer Formulierung auf der Basis von zuvor in Kolloiddispersion gebrachtem, getrocknetem und zu Pulver reduziertem Bariumsulfat zur Herstellung eines schmerzstillenden Mittels.

11. Verwendung einer aktiven Substanz, erzeugt anhand einer Formulierung auf der Basis von zuvor in Kolloiddispersion gebrachtem, getrocknetem und zu Pulver reduziertem Bariumsulfat zur Herstellung einer antipruritischen Zusammensetzung.

12. Verwendung einer aktiven Substanz, erzeugt anhand einer Formulierung auf der Basis von zuvor in Kolloiddispersion gebrachtem, getrocknetem und zu Pulver reduziertem Bariumsulfat zur Herstellung eines Medikaments zum Behandeln von Allergien.

13. Verwendung einer aktiven Substanz, erzeugt anhand einer Formulierung auf der Basis von zuvor in Kolloiddispersion gebrachtem, getrocknetem und zu Pulver reduziertem Bariumsulfat zur Herstellung einer antibakteriellen Zusammensetzung.

14. Verwendung einer aktiven Substanz, erzeugt anhand einer Formulierung auf der Basis von zuvor in Kolloiddispersion gebrachtem, getrocknetem und zu Pulver reduziertem Bariumsulfat zur Herstellung einer antiviralen und antimykotischen Zusammensetzung.

15. Verwendung einer aktiven Substanz, erzeugt anhand einer Formulierung auf der Basis von zuvor in Kolloiddispersion gebrachtem, getrocknetem und zu Pulver reduziertem Bariumsulfat zur Herstellung einer Zusammensetzung zur Verbesserung der Gefäßbildung der Haut.

16. Verwendung einer aktiven Substanz, erzeugt anhand einer Formulierung auf der Basis von zuvor in Kolloiddispersion gebrachtem, getrocknetem und zu Pulver reduziertem Bariumsulfat zur Herstellung einer Zusammensetzung zum Bleichen der Haut.
